# EUROPEAN PATENT APPLICATION

(11) **EP 1 500 703 A2**
(43) Date of publication of application: **26.01.2005**
(21) Application number: 04024482.4
(22) Date of filing: 27.06.2001
(51) Int. Cl.: C12N 15/12, C07K 14/705

(54) **Human gaba receptors and polynucleotides encoding the same**

(30) Priority: 27.06.2000 US 214083 P
(62) Divisional of application: 01950535.3
(71) Applicant: Lexicon Genetics Incorporated, The Woodlands, TX 77381-1160 (US)
(72) Inventor: Walke, D., Wade, Spring, TX 77389 (US); Friddle, Carl, Johan, The Woodlands, TX 77382 (US); Mathur, Brian, Nashville, TN 37203 (US); Turner, C., Alexander, Jr., The Woodlands, TX 77381 (US)
(74) Representative: Silveston, Judith

(57) **Abstract**

Novel human polynucleotide and polypeptide sequences are disclosed that can be used in therapeutic, diagnostic, and pharmacogenomic applications.

SEQUENCE LISTING

<110> LEXICON GENETICS INCORPORATED

<120> Novel Human GABA Receptors and
  Polynucleotides Encoding the Same

<130> 9091(1) EP JSvn/TJH

<150> US 60/214,083
<151> 2000-06-27

<160> 5

<170> FastSEQ for Windows Version 4.0

<210> 1
<211> 1398
<212> DNA
<213> homo sapiens

<400> 1
atgggtcctt tgaaagcttt tctcttctcc ccttttcttc tgcggagtca aagtagaggg      60
gtgaggttgg tcttcttgtt actgaccctg catttgggaa actgtgttga taaggcagat     120
gatgaagatg atgaggattt aacggtgaac aaaacctggg tcttggcccc aaaaattcat     180
gaaggagata tcacacaaat tctgaattca ttgcttcaag gctatgacaa taaacttcgt     240
ccagatatag gagtgaggcc cacrgtaatt gaaactgatg tttatgtaaa cagcattgga     300
ccagttgatc caattaatat ggaatataca atagatataa tttttgccca aacctggttt     360
gacagtcgtt taaaattcaa tagtaccatg aaagtgctta tgcttaacag taatatggtt     420
ggaaaaattt ggattcctga cactttcttc agaaactcaa gaaaatctga tgctcactgg     480
ataacaactc ctaatcgtct gcttcgaatt tggaatgatg gacgagttct gtatactcta     540
agattgacaa ttaatgcaga atgttatctt cagcttcata actttcccat ggatgaacat     600
tcctgtccac tggaattttc aagctatgga taccctaaaa atgaaattga gtataagtgg     660
aaaaagccct ccgtagaagt ggctgatcct aaatactgga gattatatca gtttgcattt     720
gtagggttac ggaactcaac tgaaatcact cacacgatct cwggggatta kgttatcatg     780
acaatttttt ttgacctgag cagaagaatg ggatatttca ctattcagac ctacattcca     840
tgcattctga cagttgttct ttcttgggtg tctttttgga tcaataaaga tgcagtgcct     900
gcaagaacat cgttgggtat cactacagtt ctgactatga caaccctgag tacaattgcc     960
aggaagtctt tacctaaggt ttcttatgtg actgcgatgg atctctttgt ttctgtttgt    1020
ttcatttttg tttttgcagc cttgatggaa tatggaacct tgcattattt taccagcaac    1080
caaaaaggaa agactgctac taaagacaga aagctaaaaa ataaagcctc gatgactcct    1140
ggtctccatc ctggatccac tctgattcca atgaataata tttctgtgcc gcaagaagat    1200
gattatgggt atcagtgttt ggagggcaaa gattgtgcca gcttcttctg ttgctttgaa    1260
gactgcagaa caggatcttg gagggaagga aggatacaca tacgcattgc caaaattgac    1320
tcttattcta gaatattttt cccaaccgct tttgccctgt tcaacttggt ttattgggtt    1380
ggctatcttt acttataa                                                  1398

<210> 2
<211> 465
<212> PRT
<213> homo sapiens

<400> 2
Met Gly Pro Leu Lys Ala Phe Leu Phe Ser Pro Phe Leu Leu Arg Ser
 1               5                  10                  15
Gln Ser Arg Gly Val Arg Leu Val Phe Leu Leu Leu Thr Leu His Leu
            20                  25                  30
Gly Asn Cys Val Asp Lys Ala Asp Asp Glu Asp Asp Glu Asp Leu Thr
        35                  40                  45
Val Asn Lys Thr Trp Val Leu Ala Pro Lys Ile His Glu Gly Asp Ile
    50                  55                  60
Thr Gln Ile Leu Asn Ser Leu Leu Gln Gly Tyr Asp Asn Lys Leu Arg
65                  70                  75                  80
Pro Asp Ile Gly Val Arg Pro Thr Val Ile Glu Thr Asp Val Tyr Val
                85                  90                  95
Asn Ser Ile Gly Pro Val Asp Pro Ile Asn Met Glu Tyr Thr Ile Asp

            100                 105                 110
Ile Ile Phe Ala Gln Thr Trp Phe Asp Ser Arg Leu Lys Phe Asn Ser
        115                 120                 125
Thr Met Lys Val Leu Met Leu Asn Ser Asn Met Val Gly Lys Ile Trp
    130                 135                 140
Ile Pro Asp Thr Phe Phe Arg Asn Ser Arg Lys Ser Asp Ala His Trp
145                 150                 155                 160
Ile Thr Thr Pro Asn Arg Leu Leu Arg Ile Trp Asn Asp Gly Arg Val
                165                 170                 175
Leu Tyr Thr Leu Arg Leu Thr Ile Asn Ala Glu Cys Tyr Leu Gln Leu
            180                 185                 190
His Asn Phe Pro Met Asp Glu His Ser Cys Pro Leu Glu Phe Ser Ser
        195                 200                 205
Tyr Gly Tyr Pro Lys Asn Glu Ile Glu Tyr Lys Trp Lys Lys Pro Ser
    210                 215                 220
Val Glu Val Ala Asp Pro Lys Tyr Trp Arg Leu Tyr Gln Phe Ala Phe
225                 230                 235                 240
Val Gly Leu Arg Asn Ser Thr Glu Ile Thr His Thr Ile Ser Gly Asp
                245                 250                 255
Tyr Val Ile Met Thr Ile Phe Phe Asp Leu Ser Arg Arg Met Gly Tyr
            260                 265                 270
Phe Thr Ile Gln Thr Tyr Ile Pro Cys Ile Leu Thr Val Val Leu Ser
        275                 280                 285
Trp Val Ser Phe Trp Ile Asn Lys Asp Ala Val Pro Ala Arg Thr Ser
    290                 295                 300
Leu Gly Ile Thr Thr Val Leu Thr Met Thr Thr Leu Ser Thr Ile Ala
305                 310                 315                 320
Arg Lys Ser Leu Pro Lys Val Ser Tyr Val Thr Ala Met Asp Leu Phe
                325                 330                 335
Val Ser Val Cys Phe Ile Phe Val Phe Ala Ala Leu Met Glu Tyr Gly
            340                 345                 350
Thr Leu His Tyr Phe Thr Ser Asn Gln Lys Gly Lys Thr Ala Thr Lys
        355                 360                 365
Asp Arg Lys Leu Lys Asn Lys Ala Ser Met Thr Pro Gly Leu His Pro
    370                 375                 380
Gly Ser Thr Leu Ile Pro Met Asn Asn Ile Ser Val Pro Gln Glu Asp
385                 390                 395                 400
Asp Tyr Gly Tyr Gln Cys Leu Glu Gly Lys Asp Cys Ala Ser Phe Phe
                405                 410                 415
Cys Cys Phe Glu Asp Cys Arg Thr Gly Ser Trp Arg Glu Gly Arg Ile
            420                 425                 430
His Ile Arg Ile Ala Lys Ile Asp Ser Tyr Ser Arg Ile Phe Phe Pro
        435                 440                 445
Thr Ala Phe Ala Leu Phe Asn Leu Val Tyr Trp Val Gly Tyr Leu Tyr
    450                 455                 460
Leu
465

<210> 3
<211> 771
<212> DNA
<213> homo sapiens

<400> 3
   atgggtcctt tgaaagcttt tctcttctcc ccttttcttc tgcggagtca aagtagaggg 60
   gtgaggttgg tcttcttgtt actgaccctg catttgggaa actgtgttga taaggcagat 120
   gatgaagatg atgaggattt aacggtgaac aaaacctggg tcttggcccc aaaaattcat 180
   gaaggagata tcacacaaat tctgaattca ttgcttcaag gctatgacaa taaacttcgt 240
   ccagatatag gagtgaggcc cacagtaatt gaaactgatg tttatgtaaa cagcattgga 300
   ccagttgatc caattaatat ggaatataca atagatataa tttttgccca aacctggttt 360
   gacagtcgtt taaaattcaa tagtaccatg aaagtgctta tgcttaacag taatatggtt 420
   ggaaaaattt ggattcctga cactttcttc agaaactcaa gaaaatctga tgctcactgg 480
   ataacaactc ctaatcgtct gcttcgaatt tggaatgatg gacgagttct gtatactcta 540
   agattgacaa ttaatgcaga atgttatctt cagcttcata actttcccat ggatgaacat 600
   tcctgtccac tggaattttc aagctatgga taccctaaaa atgaaattga gtataagtgg 660
   aaaaagccct ccgtagaagt ggctgatcct aaatactgga gattatatca gtttgcattt 720
   gtagggttac ggaactcaac tgaaatcact cacacgatct ctggggatta g 771

<210> 4
<211> 256
<212> PRT
<213> homo sapiens

<400> 4
Met Gly Pro Leu Lys Ala Phe Leu Phe Ser Pro Phe Leu Leu Arg Ser
1               5                   10                  15
Gln Ser Arg Gly Val Arg Leu Val Phe Leu Leu Leu Thr Leu His Leu
            20                  25                  30
Gly Asn Cys Val Asp Lys Ala Asp Asp Glu Asp Asp Glu Asp Leu Thr
        35                  40                  45
Val Asn Lys Thr Trp Val Leu Ala Pro Lys Ile His Glu Gly Asp Ile
    50                  55                  60
Thr Gln Ile Leu Asn Ser Leu Leu Gln Gly Tyr Asp Asn Lys Leu Arg
65                  70                  75                  80
Pro Asp Ile Gly Val Arg Pro Thr Val Ile Glu Thr Asp Val Tyr Val
                85                  90                  95
Asn Ser Ile Gly Pro Val Asp Pro Ile Asn Met Glu Tyr Thr Ile Asp
            100                 105                 110
Ile Ile Phe Ala Gln Thr Trp Phe Asp Ser Arg Leu Lys Phe Asn Ser
        115                 120                 125
Thr Met Lys Val Leu Met Leu Asn Ser Asn Met Val Gly Lys Ile Trp
    130                 135                 140
Ile Pro Asp Thr Phe Phe Arg Asn Ser Arg Lys Ser Asp Ala His Trp
145                 150                 155                 160
Ile Thr Thr Pro Asn Arg Leu Leu Arg Ile Trp Asn Asp Gly Arg Val
                165                 170                 175
Leu Tyr Thr Leu Arg Leu Thr Ile Asn Ala Glu Cys Tyr Leu Gln Leu
            180                 185                 190
His Asn Phe Pro Met Asp Glu His Ser Cys Pro Leu Glu Phe Ser Ser
        195                 200                 205
Tyr Gly Tyr Pro Lys Asn Glu Ile Glu Tyr Lys Trp Lys Lys Pro Ser
    210                 215                 220
Val Glu Val Ala Asp Pro Lys Tyr Trp Arg Leu Tyr Gln Phe Ala Phe
225                 230                 235                 240
Val Gly Leu Arg Asn Ser Thr Glu Ile Thr His Thr Ile Ser Gly Asp
                245                 250                 255

<210> 5
<211> 1568
<212> DNA
<213> homo sapiens

<400> 5
   ggtgcactgc ctttccacac tctcccttct gtactcagcc agctgctgct gaggtgggag      60
   gaaaagtcct ggctgggaga attgagctag tgcagcacac gtaaaaagcc gattccgatg     120
   ggtcctttga aagcttttct cttctcccct tttcttctgc ggagtcaaag tagaggggtg     180
   aggttggtct tcttgttact gaccctgcat ttgggaaact gtgttgataa ggcagatgat     240
   gaagatgatg aggatttaac ggtgaacaaa acctgggtct tggccccaaa aattcatgaa     300
   ggagatatca cacaaattct gaattcattg cttcaaggct atgacaataa acttcgtcca     360
   gatataggag tgaggcccac rgtaattgaa actgatgttt atgtaaacag cattggacca     420
   gttgatccaa ttaatatgga atatacaata gatataattt ttgcccaaac ctggtttgac     480
   agtcgtttaa aattcaatag taccatgaaa gtgcttatgc ttaacagtaa tatggttgga     540
   aaaatttgga ttcctgacac tttcttcaga aactcaagaa aatctgatgc tcactggata     600
   acaactccta atcgtctgct tcgaatttgg aatgatggac gagttctgta tactctaaga     660
   ttgacaatta atgcagaatg ttatcttcag cttcataact ttcccatgga tgaacattcc     720
   tgtccactgg aattttcaag ctatggatac cctaaaaatg aaattgagta taagtggaaa     780
   aagccctccg tagaagtggc tgatcctaaa tactggagat tatatcagtt tgcatttgta     840
   gggttacgga actcaactga aatcactcac acgatctcwg gggattakgt tatcatgaca     900
   attttttttg acctgagcag aagaatggga tatttcacta ttcagaccta cattccatgc     960
   attctgacag ttgttctttc ttgggtgtct ttttggatca ataaagatgc agtgcctgca    1020
   agaacatcgt tgggtatcac tacagttctg actatgacaa ccctgagtac aattgccagg    1080
   aagtctttac ctaaggtttc ttatgtgact gcgatggatc tctttgtttc tgtttgtttc    1140
   atttttgttt ttgcagcctt gatggaatat ggaaccttgc attattttac cagcaaccaa    1200
   aaaggaaaga ctgctactaa agacagaaag ctaaaaaata aagcctcgat gactcctggt    1260
   ctccatcctg gatccactct gattccaatg aataatattt ctgtgccgca agaagatgat    1320
   tatgggtatc agtgtttgga gggcaaagat tgtgccagct tcttctgttg ctttgaagac    1380
   tgcagaacag gatcttggag ggaaggaagg atacacatac gcattgccaa aattgactct 1440
   tattctagaa tatttttccc aaccgctttt gccctgttca acttggttta ttgggttggc 1500
   tatctttact tataaaatct acttcataag caaaaatcaa aagaagtctt gacttaaatt 1560
   tcaagtag 1568

## Description

The present application claims the benefit of U.S. Provisional Application Number 60/214,083 which was filed on June 27, 2000 and is herein incorporated by reference in its entirety.

### 1. INTRODUCTION

The present invention relates to the discovery, identification, and characterization of novel human polynucleotides encoding proteins that share sequence similarity with animal gamma-amino butyric acid (GABA) receptor subunits. The invention encompasses the described polynucleotides, host cell expression systems, the encoded proteins, fusion proteins, polypeptides and peptides, antibodies to the encoded proteins and peptides, and genetically engineered animals that either lack or over express the disclosed polynucleotides, antagonists and agonists of the proteins, and other compounds that modulate the expression or activity of the proteins encoded by the disclosed polynucleotides that can be used for diagnosis, drug screening, clinical trial monitoring, the treatment of diseases and disorders, and cosmetic or nutriceutical applications.

### 2. BACKGROUND OF THE INVENTION

Membrane proteins play important roles as, *inter alia,* cell surface markers, receptors, and mediators of signal transduction. GABA receptors bind potent inhibitory neurotransmitters and this interaction serves as a target for a variety of pharmaceutically active agents such as benzodiazepines, barbiturates, and alcohol.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with membrane receptors such as, but not limited to, human and other mammalian GABA receptors.

The novel human nucleic acid sequences described herein, encode alternative proteins/open reading frames (ORFs) of 465 and 256 amino acids in length (see SEQ ID NOS: 2 and 4 respectively).

The invention also encompasses agonists and antagonists of the described NHPs, including small molecules, large molecules, mutant NHPs, or portions thereof, that compete with native NHP, peptides, and antibodies, as well as nucleotide sequences that can be used to inhibit the expression of the described NHPs (e.g., antisense and ribozyme molecules, and gene or regulatory sequence replacement constructs) or to enhance the expression of the described NHP polynucleotides (e.g., expression constructs that place the described polynucleotide under the control of a strong promoter system), and transgenic animals that express a NHP transgene, or "knock-outs" (which can be conditional) that do not express a functional NHP. Knock-out mice can be produced in several ways, one of which involves the use of mouse embryonic stem cells ("ES cells") lines that contain gene trap mutations in a murine homolog of at least one of the described NHPs. When the unique NHP sequences described in SEQ ID NOS:1-5 are "knocked-out" they provide a method of identifying phenotypic expression of the particular gene as well as a method of assigning function to previously unknown genes.

Additionally, the unique NHP sequences described in SEQ ID NOS:1-5 are useful for the identification of coding sequence and the mapping a unique gene to a particular chromosome. Identification of actual exon splice junctions as opposed to those predicted bioinformatically.

Additionally, the unique NHP sequences described in SEQ ID NOS:1-5 are useful for the identification of coding sequence and mapping a unique gene to a particular chromosome as well as identification of the actual biologically relevant exon splice junctions. as opposed to those that might have been predicted from sequence alone.

Further, the present invention also relates to processes for identifying compounds that modulate, i.e., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of the described NHP ORFs that encode the described NHP amino acid sequences. SEQ ID NO: 5 describes an NHP ORF and flanking sequence.

### 5. DETAILED DESCRIPTION OF THE INVENTION

The NHPs, described for the first time herein, are novel proteins that are expressed in, *inter alia,* human cell lines, human brain, pituitary, cerebellum, lymph node, adipose, esophagus, cervix, rectum, pericardium, and hypothalamus cells.

The present invention encompasses the nucleotides presented in the Sequence Listing, host cells expressing such nucleotides, the expression products of such nucleotides, and: (a) nucleotides that encode mammalian homologs of the described genes, including the specifically described NHPs, and the NHP products; (b) nucleotides that encode one or more portions of the NHPs that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of an NHP, or one of its domains (*e.g.*, a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the

### Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF) that hybridizes to a complement of a DNA sequence presented in the Sequence Listing under highly stringent conditions, *e.g.,* hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. *et al*., eds., 1989, Current Protocols in Molecular Biology, Vol. I, Green Publishing Associates; Inc., and John Wiley & sons, Inc., New York, at p. 2.10.3) and encodes a functionally equivalent gene product. Additionally contemplated are any nucleotide sequences that hybridize to the complement of a DNA sequence that encodes and expresses an amino acid sequence presented in the Sequence Listing under moderately stringent conditions, e.g., washing in 0.2xSSC/0.1% SDS at 42°C (Ausubel et al., 1989, *supra),* yet still encodes a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding NHP ORFs, or their functional equivalents, encoded by polynucleotide sequences that are about 99, 95, 90, or about 85 percent similar or identical to corresponding regions of the nucleotide sequences of the Sequence Listing (as measured by BLAST sequence comparison analysis using, for example, the GCG sequence analysis package (Madison, Wisconsin) using standard default settings).

The invention also includes nucleic acid molecules, preferably DNA molecules, that hybridize to, and are therefore the complements of, the described NHP gene nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-5 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-5, or an amino acid sequence encoded thereby. Methods for attaching biopolymers to, or synthesizing biopolymers on, solid support matrices, and conducting binding studies thereon are disclosed in, *inter alia*, U.S. Patent Nos. 5,700,637, 5,556,752, 5,744,305, 4,631,211, 5,445,934, 5,252,743, 4,713,326, 5,424,186, and 4,689,405 the disclosures of which are herein incorporated by reference in their entirety.

Addressable arrays comprising sequences first disclosed in SEQ ID NOS:1-5 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides and more preferably 25 nucleotides from the sequences first disclosed in SEQ ID NOS:1-5.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-5 provides detailed information about transcriptional changes involved in a specific pathway, potentially leading to the identification of novel components or gene functions that manifest themselves as novel phenotypes.

Probes consisting of sequences first disclosed in SEQ ID NOS:1-5 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-5 can be utilized in microarrays or other assay formats, to screen collections of genetic material from patients who have a particular medical condition. These investigations can also be carried out using the sequences first disclosed in SEQ ID NOS:1-5 *in silico* and by comparing previously collected genetic databases and the disclosed sequences using computer software known to those in the art.

Thus the sequences first disclosed in SEQ ID NOS:1-5 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-5. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (e.g., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relative to one or more additional sequence(s) or one or more restriction sites present in the disclosed sequence.

For oligonucleotide probes, highly stringent conditions may refer, *e.g.*, to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. *15*:6625-6641). The oligonucleotide is a 2'-0-methylribonucleotide (Inoue *et al*., 1987, Nucl. Acids Res. *15*:6131-6148), or a chimeric RNA-DNA analogue (Inoue *et al.,* 1987, FEBS Lett. *215*:327-330). Alternatively, double stranded RNA can be used to disrupt the expression,and function of a targeted NHP.

Oligonucleotides of the invention can be synthesized by standard methods known in the art, *e.g.* by use of an automated DNA synthesizer (such as are commercially available from Biosearch, Applied Biosystems, etc.). As examples, phosphorothioate oligonucleotides can be synthesized by the method of Stein *et al*. (1988, Nucl. Acids Res. *16*:3209), and methylphosphonate oligonucleotides can be prepared by use of controlled pore glass polymer supports (Sarin *et al*., 1988, Proc. Natl. Acad. Sci. U.S.A. *85*:7448-7451), etc.

Low stringency conditions are well known to those of skill in the art, and will vary predictably depending on the specific organisms from which the library and the labeled sequences are derived. For guidance regarding such conditions see, for example, Sambrook *et al.*, 1989, Molecular Cloning, A Laboratory Manual (and periodic updates thereof), Cold Springs Harbor Press, N.Y.; and Ausubel *et al*., 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

Further, a NHP gene homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene.

The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e.*, one known, or suspected, to express a NHP gene). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g.*, Sambrook *et al*., 1989, *supra.*

A cDNA encoding a mutant NHP gene can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of or known to carry a mutant NHP allele (*e.g.*, a person manifesting a NHP-associated phenotype such as, for example, obesity, vision disorders, high blood pressure, depression, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP gene sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue can be expressed and screened using standard antibody screening techniques in conjunction with antibodies raised against a normal NHP product, as described below. (For screening techniques, see, for example, Harlow, E. and Lane, eds., 1988, "Antibodies: A Laboratory Manual", Cold Spring Harbor Press, Cold Spring Harbor, NY.)

Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function (*e.g.*, as a result of a missense or a frameshift mutation), polyclonal antibodies to a NHP are likely to cross-react with a corresponding mutant NHP gene product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e.*, antisense); (b) DNA expression vectors that contain any of the foregoing NHP coding sequences operatively associated with a regulatory element that directs the expression of the coding sequences (for example, baculo virus as described in U.S. Patent No. 5,869,336 herein incorporated by reference); (c) genetically engineered host cells that contain any of the foregoing NHP coding sequences operatively associated with a regulatory element that directs the expression of the coding sequences in the host cell; and (d) genetically engineered host cells that express an endogenous NHP gene under the control of an exogenously introduced regulatory element (*i.e.*, gene activation). As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of the NHP, as well as compounds or nucleotide constructs that inhibit expression of a NHP gene (transcription factor inhibitors, antisense and ribozyme molecules, or gene or regulatory sequence replacement constructs), or promote the expression of a NHP (*e.g.*, expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NHP in the body. The use of engineered host cells and/or animals may offer an advantage in that such systems allow not only for the identification of compounds that bind to the endogenous receptor for an NHP, but can also identify compounds that trigger NHP-mediated activities or pathways.

Finally, the NHP products can be used as therapeutics. For example, soluble derivatives such as NHP peptides/domains corresponding to the NHPs, NHP fusion protein products (especially NHP-Ig fusion proteins, i.e., fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics the NHP could activate or effectively antagonize the endogenous NHP receptor. Nucleotide constructs encoding such NHP products can be used to genetically engineer host cells to express such products in vivo; these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEQUENCES

The cDNA sequences and the corresponding deduced amino acid sequences of the described NHPs are presented in the Sequence Listing. The NHP nucleotides were obtained from clustered human sequences and cDNA isolated from a human brain cDNA library as well as cDNAs made from fetal brain, adult brain, pituitary gland, and cerebellum mRNA. The described sequences share structural similarity with GABA receptor proteins, and particularly GABA A receptor gamma-1, -2, and -3, -4, -5, and -6 subunits. Because of their medical relevance, GABA receptors have been subject to considerable scientific scrutiny as shown in U.S. Application No. 09/183,253 (corresponding to W09942580A2), herein incorporated by reference, which describes a variety of uses, assays, and applications that can be applied to the presently described NHPs.

An additional application of the described novel human polynucleotide sequences is their use in the molecular mutagenesis/evolution of proteins that are at least partially encoded by the described novel sequences using, for example, polynucleotide shuffling or related methodologies. Such approaches are described in U.S. Patents Nos. 5,830,721 and 5,837,458 which are herein incorporated by reference in their entirety.

NHP gene products can also be expressed in transgenic animals. Animals of any species, including, but not limited to, worms, mice, rats, rabbits, guinea pigs, pigs, micro-pigs, birds, goats, and non-human primates, *e.g.*, baboons, monkeys, and chimpanzees may be used to generate NHP transgenic animals.

Any technique known in the art may be used to introduce a NHP transgene into animals to produce the founder lines of transgenic animals. Such techniques include, but are not limited to pronuclear microinjection (Hoppe, P.C. and Wagner, T.E., 1989, U.S. Pat. No. 4,873,191); retrovirus mediated gene transfer into germ lines (Van der Putten et al., 1985, Proc. Natl. Acad. Sci., USA *82*:6148-6152); gene targeting in embryonic stem cells (Thompson et *al.,* 1989, Cell *56*:313-321); electroporation of embryos (Lo, 1983, Mol Cell. Biol. *3*:1803-1814); and sperm-mediated gene transfer (Lavitrano *et al*., 1989, Cell *57*:717-723); etc. For a review of such techniques, see Gordon, 1989, Transgenic Animals, Intl. Rev. Cytol. *115*:171-229, which is incorporated by reference herein in its entirety.

The present invention provides for transgenic animals that carry the NHP transgene in all their cells, as well as animals which carry the transgene in some, but not all their cells, *i.e.*, mosaic animals or somatic cell transgenic animals. The transgene may be integrated as a single transgene or in concatamers, *e.g.*, head-to-head tandems or head-to-tail tandems. The transgene may also be selectively introduced into and activated in a particular cell type by following, for example, the teaching of Lasko *et al.*, 1992, Proc. Natl. Acad. Sci. USA *89*:6232-6236. The regulatory sequences required for such a cell-type specific activation will depend upon the particular cell type of interest, and will be apparent to those of skill in the art.

When it is desired that a NHP transgene be integrated into the chromosomal site of the endogenous NHP gene, gene targeting is preferred. Briefly, when such a technique is to be utilized, vectors containing some nucleotide sequences homologous to the endogenous NHP gene are designed for the purpose of integrating, via homologous recombination with chromosomal sequences, into and disrupting the function of the nucleotide sequence of the endogenous NHP gene *(i.e.,* "knockout" animals).

The transgene can also be selectively introduced into a particular cell type, thus inactivating the endogenous NHP gene in only that cell type, by following, for example, the teaching of Gu *et al.*, 1994, Science, 265:103-106. The regulatory sequences required for such a cell-type specific inactivation will depend upon the particular cell type of interest, and will be apparent to those of skill in the art.

Once transgenic animals have been generated, the expression of the recombinant NHP gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include but are not limited to Northern blot analysis of tissue samples obtained from the animal, *in situ* hybridization analysis, and RT-PCR. Samples of NHP gene-expressing tissue, may also be evaluated immunocytochemically using antibodies specific for the NHP transgene product.

### 5.2 NHPS AND NHP POLYPEPTIDES

NHPs, polypeptides, peptide fragments, mutated, truncated, or deleted forms of the NHPs, and/or NHP fusion proteins can be prepared for a variety of uses. These uses include but are not limited to the generation of antibodies, as reagents in diagnostic assays, the identification of other cellular gene products related to a NHP, as reagents in assays for screening for compounds that can be used as pharmaceutical reagents useful in the therapeutic treatment of mental, biological, or medical disorders and diseases. Given the similarity information and expression data, the described NHPs can be targeted (by drugs, oligos, antibodies, etc,) in order to treat disease, or to therapeutically augment the efficacy of therapeutic agents.

The Sequence Listing discloses the amino acid sequences encoded by the described NHP genes. The NHPs typically display initiator methionines in DNA sequence contexts consistent with a translation initiation site, and a signal sequence characteristic of membrane or secreted proteins.

The NHP amino acid sequences of the invention include the amino acid sequences presented in the Sequence Listing as well as analogues and derivatives thereof. Further, corresponding NHP homologues from other species are encompassed by the invention. In fact, any NHP protein encoded by the NHP nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell *et al*. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (e.g., proteolytic activity, ion flux, tyrosine phosphorylation, transport, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, the NHP peptide or polypeptide is thought to be membrane protein, the hydrophobic regions of the protein can be excised and the resulting soluble peptide or polypeptide can be recovered from the culture media. Such expression systems also encompass engineered host cells that express a NHP, or functional equivalent, *in situ*. Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, *e.g.*, in drug screening assays.

The expression systems that can be used for purposes of the invention include but are not limited to microorganisms such as bacteria (*e.g., E. coli*, *B. subtilis*) transformed with recombinant bacteriophage DNA, plasmid DNA or cosmid DNA expression vectors containing NHP nucleotide sequences; yeast (*e.g., Saccharomyces, Pichia*) transformed with recombinant yeast expression vectors containing NHP nucleotide sequences; insect cell systems infected with recombinant virus expression vectors (*e.g.*, baculovirus) containing NHP sequences; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing NHP nucleotide sequences; or mammalian cell systems (*e.g.*, COS, CHO, BHK, 293, 3T3) harboring recombinant expression constructs containing promoters derived from the genome of mammalian cells (*e.g.*, metallothionein promoter) or from mammalian viruses (*e.g.*, the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the *E. coli* expression vector pUR278 (Ruther *et al*., 1983, EMBO J. *2*:1791), in which a NHP coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. *264*:5503-5509); and the like. pGEX vectors (Pharmacia or American Type Culture Collection) can also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NHP gene coding sequence may be cloned individually into non-essential regions (for example the polyhedrin gene) of the virus and placed under control of an AcNPV promoter (for example the polyhedrin promoter). Successful insertion of NHP gene coding sequence will result in inactivation of the polyhedrin gene and production of non-occluded recombinant virus (*i.e.*, virus lacking the proteinaceous coat coded for by the polyhedrin gene). These recombinant viruses are then used to infect Spodoptera frugiperda cells in which the inserted gene is expressed (*e.g.*, see Smith *et al*., 1983, J. Virol. *46*:584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e*.*g*., region E1 or E3) will result in a recombinant virus that is viable and capable of expressing a NHP product in infected hosts (*e.g.*, See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter *et al.,* 1987, Methods in Enzymol. *153*:516-544).

In addition, a host cell strain may be chosen that modulates the expression of the inserted sequences, or modifies and processes the gene product in the specific fashion desired. Such modifications (*e.g.*, glycosylation) and processing (*e.g.*, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e.g.*, promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, *et al.,* 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, *et al.,* 1980, Cell *22*:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, *et al.,* 1980, Natl. Acad. Sci. USA *77*:3567; O'Hare, *et al.,* 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, *et al*., 1981, J. Mol. Biol. 150:1); and hygro, which confers resistance to hygromycin (Santerre, *et al.*, 1984, Gene 30:147).

Alternatively, any fusion protein can be readily purified by utilizing an antibody specific for the fusion protein being expressed. For example, a system described by Janknecht *et al*. allows for the ready purification of non-denatured fusion proteins expressed in human cell lines (Janknecht, *et al.,* 1991, Proc. Natl. Acad. Sci. USA *88*:8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in "Liposomes:A Practical Approach", New, RRC ed., Oxford University Press, New York and in U.S. Patents Nos. 4,594,595, 5,459,127, 5,948,767 and 6,110,490 and their respective disclosures which are herein incorporated by reference in their entirety. Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain (see generally U.S. applications Ser. No. 60/111,701 and 60/056,713, both of which are herein incorporated by reference, for examples of such transducing sequences) to facilitate passage across cellular membranes and can optionally be engineered to include nuclear localization sequences.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP gene product. Additionally, such antibodies can be used in conjunction gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with the NHP, an NHP peptide (*e.g.*, one corresponding to a functional domain of an NHP), truncated NHP polypeptides (NHP in which one or more domains have been deleted), functional equivalents of the NHP or mutated variant of the NHP. Such host animals may include but are not limited to pigs, rabbits, mice, goats, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum*. Alternatively, the immune response could be enhanced by combination and or coupling with molecules such as keyhole limpet hemocyanin, tetanus toxoid, diphtheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor *et al*., 1983, Immunology Today *4*:72; Cole *et al.,* 1983, Proc. Natl. Acad. Sci. USA *80*:2026-2030), and the EBV-hybridoma technique (Cole *et al.,* 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison *et al*., 1984, Proc. Natl. Acad. Sci., *81*:6851-6855; Neuberger *et al*., 1984, Nature, *312*:604-608; Takeda *et al.,* 1985, Nature, *314*:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures which are herein incorporated by reference in their entirety. Also encompassed by the present invention is the use of fully humanized monoclonal antibodies as described in US Patent No. 6,150,584 and respective disclosures which are herein incorporated by reference in their entirety.

Alternatively, techniques described for the production of single chain antibodies (U.S. Patent 4,946,778; Bird, 1988, Science *242*:423-426; Huston *et al.,* 1988, Proc. Natl. Acad. Sci. USA *85*:5879-5883; and Ward *et al*., 1989, Nature *341*:544-546) can be adapted to produce single chain antibodies against NHP gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse *et al.*, 1989, Science, 246:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, *e.g.,* Greenspan & Bona, 1993, FASEB J *7(5)*:437-444; and Nissinoff, 1991, J. Immunol. *147(8)*:2429-2438). For example antibodies which bind to a NHP domain and competitively inhibit the binding of NHP to its cognate receptor can be used to generate anti-idiotypes that "mimic" the NHP and, therefore, bind and activate or neutralize a receptor. Such anti-idiotypic antibodies or Fab fragments of such anti-idiotypes can be used in therapeutic regimens involving a NHP mediated pathway.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. All cited publications, patents, and patent applications are herein incorporated by reference in their entirety.

## Claims

1. An isolated nucleic acid molecule comprising a nucleotide sequence encoding an amino acid sequence drawn from the group consisting of SEQ ID NOS: 2 and 4.

2. An isolated nucleic acid molecule comprising a nucleotide sequence that:
(a) encodes the amino acid sequence shown in SEQ ID NO: 2;
(b) hybridizes under stringent conditions to the nucleotide sequence of SEQ ID NO: 1 or the complement thereof.

3. An isolated nucleic acid molecule comprising at least 24 contiguous bases of nucleotide sequence first disclosed in SEQ ID NO: 1.

4. An isolated nucleic acid molecule comprising at least 24 contiguous bases of nucleotide sequence first disclosed in SEQ ID NO: 3.

5. A polypeptide encoded by a nucleic acid as claimed in any one of claims 1 to 4.

6. An isolated polypeptide comprising at least about 12 amino acids in a sequence first disclosed in SEQ ID NO: 2 or SEQ ID NO: 4.

7. An antibody that specifically recognises a polypeptide as claimed in claim 5 or claim 6, or an epitope-binding fragment or a derivative of said antibody, for example, a polyclonal or monoclonal antibody, a single chain antibody, a Fab fragment, a F(ab')₂, a fragment produce by a Fab expression library, or an anti-idiotypic (anti-Id) antibody.

8. A host cell that comprises a nucleic acid as claimed in any one of claims 1 to 4 and is capable of expressing the polypeptide encoded by said nucleic acid, optionally on the surface of the cell.

9. A non-human transgenic animal that is capable of expressing a nucleic acid as claimed in any one of claims 1 to 4, or that is an optionally conditional "knock-out" animal that does not express a polypeptide as claimed in claim 5 or claim 6.

10. Use of nucleic acid as claimed in any one of claims 1 to 4, a polypeptide as claimed in claim 5 or claim 6, an antibody as claimed in claim 7, a host cell as claimed in claim 8 or an animal as claimed in claim 9, in a method for screening compounds for the ability to modulate the expression of a nucleic acid as claimed in any one of claims 1 to 4 or a polypeptide as claimed in claim 5 or claim 6, or that modulate the activity of a polypeptide as claimed in claim 5 or claim 6, for example, for high throughput screening of combinatorial libraries.
